(19) Europäisches Patentamt
European Patent Office
Office européen des brevets

(11) EP 0 802 189 B1

(12) EUROPÄISCHE PATENTSCHRIFT

(45) Veröffentlichungstag und Bekanntmachung des Hinweises auf die Patenterteilung:
**24.03.2004 Patentblatt 2004/13**

(51) Int Cl.[7]: **C07D 213/81**, C07D 239/42, C07D 241/24, C07D 253/06, B01J 31/24

(21) Anmeldenummer: **97105060.4**

(22) Anmeldetag: **25.03.1997**

# (54) Verfahren zur Herstellung von Arylamiden heteroaromatischer Carbonsäuren

Process of preparation of Arylamides heteroaromatic Carboxylic acids

Procédé pour la préparation des arylamides des acides carboxyliques hétéroaromatiques

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **28.03.1996 CH 80696**

(43) Veröffentlichungstag der Anmeldung:
**22.10.1997 Patentblatt 1997/43**

(73) Patentinhaber: **Lonza AG**
**3930 Visp (CH)**

(72) Erfinder:
- **Roduit, Jean-Paul, Dr.**
  **3979 Grône (Kanton Wallis) (CH)**
- **Kalbermatten, Georges**
  **3938 Ausserberg (Kanton Wallis) (CH)**

(74) Vertreter: **Winter, Brandl, Fürniss, Hübner, Röss, Kaiser, Polte Partnerschaft**
**Patent- und Rechtsanwaltskanzlei**
**Alois-Steinecker-Strasse 22**
**85354 Freising (DE)**

(56) Entgegenhaltungen:
**EP-A- 0 053 011**
**EP-A- 0 282 266**
**EP-A- 0 282 658**
**EP-A- 0 447 004**
**EP-A- 0 627 422**
**EP-A- 0 646 590**

- **A. SCHOENBERG ET AL.: "Palladium-catalyzed Amidation of Aryl, Heterocyclic, and Vinylic Halides" JOURNAL OF ORGANIC CHEMISTRY, Bd. 39, Nr. 23, 1974, Seiten 3327-3331, XP000673766 COLUMBUS OHIO**
- **RYO TAKEUCHI ET AL.: "Palladium-catalyzed carbonylation of N-heteroaromatic chloride" JOURNAL OF MOLECULAR CATALYSIS, Bd. 66, Nr. 3, 1991, Seiten 277-288, XP002037142 THE NETHERLANDS**
- **YEHOSHUA BEN-DAVID ET AL.: "Chelate-Assisted, Pd-Catalyzed Efficient Carbonylation of Aryl Chlorides" JOURNAL OF AMERICAN CHEMICAL SOCIETY, Bd. 111, Nr. 23, 1989, Seiten 8742-8744, XP002037143 COLUMBUS OHIO**
- **PATENT ABSTRACTS OF JAPAN vol. 11, no. 369 (C-461) [2816], 2. Dezember 1987 & JP 62 142161 A (MITSUI TOATSU CHEM. INC.)**
- **LORENZO TESTAFERI: "the reactionof some halogenated pyridines with methoxide and methanethiolate ions in dimethylformamide" TETRAHEDRON, Bd. 41, Nr. 7, 1985, Seiten 1373-1384, XP002081893 Great Britain**

EP 0 802 189 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft ein Verfahren zur Herstellung von Arylamiden heteroaromatischer Carbonsäuren durch Umsetzung von heteroaromatischen Halogenverbindungen mit Kohlenmonoxid und aromatischen Aminen in Gegenwart eines Katalysators und einer Base.
Die erfindungsgemäss herstellbaren Amide besitzen die allgemeine Formel

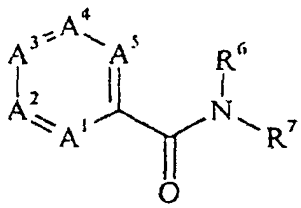

I.

Hierin bedeuten

$A^1$ Stickstoff oder $CR^1$,
$A^2$ Stickstoff oder $CR^2$,
$A^3$ Stickstoff oder $CR^3$,
$A^4$ Stickstoff oder $CR^4$,
und $A^5$ Stickstoff oder $CR^5$,
mit der Massgabe dass wenigstens eines der Ringglieder $A^1$ bis $A^5$ Stickstoff ist und nicht zwei Stickstoffatome unmittelbar miteinander verbunden sind,
$R^1$ bis $R^5$, soweit vorhanden, unabhängig voneinander Wasserstoff, $C_{1-4}$-Alkyl oder Aryl, wobei jedoch einer der Substituenten $R^1$ bis $R^5$ eine Gruppe der Formel -OR ist, in welcher R ein gegebenenfalls substituierter aromatischer oder heteroaromatischer Rest ist,
$R^6$ Wasserstoff oder $C_{1\ 4}$-Alkyl
und $R^7$ einen gegebenenfalls substituierten aromatischen oder heteroaromatischen Rest Insbesondere gehören hierzu die Arylamide der Pyridin-, Pyrimidin-, Pyrazin- und 1,3,5-Triazincarbonsäuren.

Zahlreiche Verbindungen dieser Struktur, insbesondere solche, bei denen einer der Substituenten $R^1$ bis $R^5$ eine Aryloxygruppe (-OR) in Nachbarschaft zu einem Ringstickstoffatom ist, sind wichtige Herbizide (WO-A 94/27974, EP-A 0 053 011, EP-A 0 447 004). Die Synthese dieser bekannten Verbindungen geht üblicherweise von den entsprechenden Carbonsäuren oder Carbonsäurederivaten (Säurechloride, Ester, Nitrile) aus. Diese sind jedoch oft schwer zugänglich und deshalb teuer.
Aufgabe der vorliegenden Erfindung war daher, ein alternatives Verfahren zur Verfügung zu stellen, welches von leichter zugänglichen Edukten ausgeht.
Erfindungsgemäss wird diese Aufgabe durch die Verfahren nach Patentansprüchen 1 und 13 gelöst.
Es wurde gefunden, dass Halogenverbindungen der allgemeinen Formel

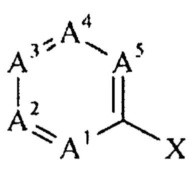

II,

worin $A^1$ bis $A^5$ die oben genannten Bedeutungen haben und X Chlor, Brom oder Iod ist, mit Kohlenmonoxid und einem primären oder sekundären Amin der allgemeinen Formel

$$R^6\text{-NH-}R^7 \qquad \text{III,}$$

worin $R^6$ und $R^7$ die oben genannten Bedeutungen haben, in Gegenwart einer Base in guter bis fast quantitativer

Ausbeute direkt zu den gewünschten Produkten (I) reagieren, wenn als Katalysator ein Komplex von Palladium mit einem Diphosphin der allgemeinen Formel

$$R^8R^9P—[CH_2]_n—PR^{10}R^{11} \qquad IV$$

anwesend ist Es bedeuten hierin $R^8$ bis $R^{11}$ unabhängig voneinander Phenyl oder substituiertes Phenyl und *n* 3 oder 4.

Unter $C_{1-4}$-Alkyl sind hier und im folgenden alle linearen oder verzweigten primären, sekundaren oder tertiären Alkylgruppen mit bis zu 4 Kohlenstoffatomen zu verstehen Unter aromatischen oder heteroaromatischen Resten sind hier und im folgenden insbesondere mono- oder polycyclische Systeme wie beispielsweise Phenyl, Naphthyl, Biphenylyl, Anthracenyl, Furyl, Pyrrolyl, Pyrazolyl, Thiophenyl, Pyridyl, Indolyl oder Chinolinyl zu verstehen Diese können einen oder mehrere gleiche oder verschiedene Substituenten tragen, beispielsweise niedrige Alkylgruppen wie Methyl, halogenierte Alkylgruppen wie Trifluormethyl, niedrige Alkoxygruppen wie Methoxy, oder niedrige Alkylthio- (Alkansulfanyl) oder Alkansulfonylgruppen wie Methylthio oder Ethansulfonyl. Unter substituiertem Phenyl sind insbesondere Gruppen wie (*p*-) Fluorphenyl, (*p*-) Methoxyphenyl, (*p*-) Tolyl oder (*p*-) Trifluormethylphenyl zu verstehen.

Die als Ausgangsmaterial dienenden Halogenverbindungen (II) sind bekannte Verbindungen oder können analog zu bekannten Verbindungen hergestellt werden. Zahlreiche derartige Verbindungen sind beispielsweise in US-A 4 254 125 und in EP-A 0 001 187 offenbart.

Diejenigen Halogenverbindungen (II), in welchen die Gruppe der Formel -OR an ein zu einem Ringstickstoffatom benachbartes Kohlenstoffatom gebunden ist, werden vorteilhaft dadurch hergestellt, dass ein Dihalogenid der allgemeinen Formel

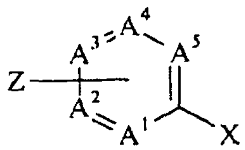

V,

worin $A^1$ bis $A^5$ die oben genannten Bedeutungen haben, mit der Massgabe, dass einer der Reste $R^1$ bis $R^5$ an einem zu einem Ringstickstoffatom benachbarten Kohlenstoffatom durch Z ersetzt ist, welches Chlor, Brom oder Iod ist, und X unabhängig davon Chlor, Brom oder Iod bedeutet, mit einer aromatischen oder heteroaromatischen Hydroxyverbindung der allgemeinen Formel

$$R\text{-}OH \qquad VI,$$

worin R die oben genannte Bedeutung hat, umgesetzt wird.

Vorzugsweise eignet sich das erfindungsgemässe Verfahren zur Herstellung solcher Amide (I), in denen $A^2$ Stickstoff ist und mit den übrigen Ringgliedern einen Pyridinring bildet Besonders bevorzugt sind dabei jene Amide (I), in denen $R^1$ eine Gruppe der Formel -OR ist, wobei R die oben genannte Bedeutung hat.

Ebenfalls bevorzugt sind solche Amide (I), in denen A' Stickstoff ist und mit den übrigen Ringgliedern einen Pyridinring bildet,
solche, in denen $A^1$ und $A^5$ Stickstoff sind und mit den übrigen Ringgliedern einen Pyrimidinring bilden,
solche, in denen $A^1$ und $A^4$ Stickstoff sind und mit den übrigen Ringgliedern einen Pyrazinring bilden,
sowie solche, in denen $A^1$, $A^3$ und $A^5$ Stickstoff sind und mit den übrigen Ringgliedern einen 1,3,5-Triazinring bilden.

Unter den vier letztgenannten Klassen sind wiederum diejenigen Amide besonders bevorzugt, in welchen $R^2$ eine Gruppe der Formel -OR ist, wobei R die oben genannte Bedeutung hat.

Unter den Amiden (I) sind ausserdem diejenigen bevorzugt, in denen R eine gegebenenfalls substituierte Phenylgruppe ist Dies gilt insbesondere für die vorstehend genannten Amide mit Pyridin-, Pyrimidin-, Pyrazin- oder 1,3,5-Triazinring, in denen $R^1$ oder $R^2$ eine Gruppe der Formel -OR ist.

Ebenfalls bevorzugt sind solche Amide, in denen $R^6$ Wasserstoff und $R^7$ eine gegebenenfalls substituierte Phenylgruppe ist

Als Halogenverbindungen (II) sind die Chlorverbindungen (X = Cl) bevorzugt

Der katalytisch wirksame Palladium-Diphosphin-Komplex wird vorteilhaft *in situ* gebildet, indem Palladium in fein verteilter elementarer Form (z. B. Palladium auf Aktivkohle), ein Pd(II) Salz (z. B. das Chlorid oder das Acetat),

oder ein geeigneter Pd(II)-Komplex (z B Dichloro-bis(triphenylphosphin)palladium(II)) mit dem Diphosphin zur Reaktion gebracht wird. Das Palladium wird vorzugsweise in einer Menge von 0,02 bis 0,2 Mol-% Pd(II) oder 0,5 bis 2 Mol-% Pd(0) (als Pd/C), jeweils bezogen auf die Halogenverbindung (II), eingesetzt. Das Diphosphin wird vorteilhaft im Uberschuss (bezogen auf Pd) eingesetzt, vorzugsweise in einer Menge von 0,2 bis 5 Mol-%, ebenfalls bezogen auf die Halogenverbindung (II)

Als Lösungsmittel können sowohl relativ unpolare, wie beispielsweise Toluol oder Xylol, als auch polare wie beispielsweise Acetonitril, Tetrahydrofuran oder *N*,*N*-Dimethylacetamid eingesetzt werden

Als Base wird vorzugsweise eine relativ schwache Base eingesetzt. Diese braucht in dem verwendeten Lösungsmittel nicht löslich zu sein Gut geeignet sind beispielsweise Carbonate wie Natriumcarbonat oder Kaliumcarbonat oder Acetate wie Natriumacetat. Mit der letztgenannten Base wurden ganz besonders gute Ergebnisse erzielt.

Die Reaktionstemperatur liegt vorzugsweise bei 80 bis 250 °C.

Der Kohlenmonoxiddruck liegt vorzugsweise bei 1 bis 50 bar.

Die folgenden Beispiele verdeutlichen die Durchführung des erfindungsgemässen Verfahrens

**Beispiel 1**

**2-Chlor-6-[3-(trifluormethyl)phenoxy]pyridin**

In 420 ml *N*,*N*-Dimethylacetamid wurden 17,45 g (690 mmol) Natriumhydrid (95%) suspendiert Bei 15 °C wurden innerhalb von 2 h 106,7 g (658 mmol) 3-(Trifluormethyl)phenol zugetropft Die so erhaltene Phenolatlösung wurde innerhalb von 2,5 h unter Stickstoff zu einer auf 90 °C erwärmten Lösung von 162,4 g (1,097 mol) 2,6-Dichlorpyridin in 330 ml *N*,*N*-Dimethylacetamid getropft. Nach weiteren 3 h Reaktionszeit wurde das Gemisch auf Raumtemperatur abgekühlt, das ausgefallene Natriumchlorid abfiltriert und das Filtrat eingeengt. Der Rückstand wurde mit Toluol und 0,1 N Salzsäure aufgenommen, die organische Phase mit gesättigter Natriumchloridlösung gewaschen und eingeengt. Der ölige Rückstand (ca 200 g) wurde im Vakuum destilliert
Ausbeute. 151,5 g (84%) farbloses Öl, Gehalt (GC) 99,8%
$n_D^{20}$ = 1,5267
MS, *m z*: 273/275, 238, 39

| | |
|---|---|
| $^1$H NMR ($CDCl_3$). δ = | 6,84 (d, *J* = 7,8 Hz, 1H), 7,07 (d, *J* = 7,8 Hz, 1H), 7,35 (m, 1H); 7,42 (m, 1H); 7,45-7,52 (m, 2H); 7,65 (t, *J* = 7,8 Hz, 1H). |
| $^{13}$C NMR ($CDCl_3$). δ = | 109,88 (CH); 118,16 (CH); 119,24 (CH); 121,67 (CH), 123,74 ($CF_3$), 124,50 (CH); 130,24 (CH); 132,21 ($CCF_3$); 141,77 (CH); 149,12 (C), 153,89 (C); 162,28 (C) |

**Beispiel 2**

**3-Chlor-2-[3-(trifluormethyl)phenoxy]pyridin**

Unter Stickstoff wurden 7,68 g Natriumhydrid-Dispersion (ca. 50 % in Mineralöl) mit Pentan gewaschen, dann wurden 100 ml *N*,*N*-Dimethylformamid zugegeben. Bei Raumtemperatur wurden innerhalb von 30 min 21,92 g (135 mmol) 3-(Trifluormethyl)phenol zugetropft Die so erhaltene Phenolatlösung wurde innerhalb von 2 h unter Stickstoff zu einer auf 120 °C erhitzten Lösung von 20,1 g (136 mmol) 2,3-Dichlorpyridin in 80 ml *N*,*N*-Dimethylformamid getropft Nach 3 h Reaktionsdauer wurde das Gemisch auf Raumtemperatur abgekühlt, das ausgefallene Natriumchlorid abfiltriert und das Filtrat eingeengt. Der Rückstand wurde mit Toluol und 0,1 N Salzsäure extrahiert, die organische Phase mit gesättigter Natriumchloridlösung gewaschen und eingeengt. Der ölige Rückstand wurde im Vakuum destilliert
Ausbeute 24,75 g (67%) farbloses Öl, Gehalt (GC) 99,7%
Sdp $_{18\ mbar}$ = 145-148 °C
$n_D^{20}$ = 1,5282
MS; *m z*: 273/275

| | |
|---|---|
| $^1$H NMR ($CDCl_3$): δ = | 6,99 (m, 1H); 7,36 (d, 1H); 7,45-7,53 (m, 3H), 7,77 (d, 1H); 8,02 (d, 1H) |
| $^{13}$C NMR ($CDCl_3$): δ = | 118,66 (CH), 119,44 (C); 119,98 (CH); 121,75 (CH); 123,78 ($CF_3$); 124,94 (CH), 130,13 (CH), 132,16 ($CCF_3$), 139,65 (CH); 145,20 (CH), 153,88 (C); 158,51 (C). |

**Beispiel 3**

***N*-(4-Fluorphenyl)-6-[3-(trifluormethyl)phenoxy]pyridin-2-carboxamid**

In einem Autoklaven wurden bei Raumtemperatur 6,84 g (25 mmol) 2-Chlor-6-[3-(trifluormethyl)phenoxy]pyridin (Gehalt 99,5%, hergestellt nach Beispiel 1), 4,17 g (37,5 mmol) 4-Fluoranilin, 2,92 g (27,5 mmol) Natriumcarbonat, 0,27 g (0,25 mmol) Palladium/Aktivkohle (10% Pd) und 0,32 g (0,75 mmol) 1,4-Bis(diphenylphosphino)butan (IV, $n$ = 4, $R^8 = R^9 = R^{10} = R^{11}$ = Phenyl) in 25 ml Xylol vorgelegt. Der Autoklav wurde mit Inertgas gespült, dann wurden 5 bar Kohlenmonoxid aufgepresst und auf 200 °C aufgeheizt. Der CO-Druck wurde auf 14,5 bar erhöht und das Gemisch 16 h bei 200 °C gerührt. Nach dem Abkühlen auf Raumtemperatur und der Druckentlastung wurde das Reaktionsgemisch mit je 50 ml Xylol und Wasser versetzt und filtriert. Die wässrige Phase wurde mit 25 ml Xylol extrahiert und die vereinigten organischen Phasen mit 30 ml Wasser gewaschen. Mittels GC wurde die Zusammensetzung der gelösten Produkte bestimmt. Gefunden wurden 92,1% Titelverbindung (Amid), 1,9% Edukt und 6,0% Nebenprodukte (3,1% sekundäres Amin, entstanden durch direkte Substitution von Cl durch das Anilin, und 2,9% 2-[3-(Trifluormethyl)phenoxy]pyridin, entstanden durch Hydrogenolyse)
Nach dem Abdestillieren des Lösungsmittels wurde das Rohprodukt (8,63 g) als gelber Feststoff erhalten
Zur Reinigung wurde das Rohprodukt aus Methylcyclohexan umkristallisiert.
Ausbeute: 6,3 g (67%) farblose Kristalle
Schmp 104-105 °C
MS, *m z*: 376 (M'), 238

$^1$H NMR ($CDCl_3$), δ = 6,99-7,04 (m, 2H); 7,17 (d, $J$ = 8,4 Hz, 1H), 7,40 (m, 1H); 7,46-7,51 (m, 2H); 7,55-7,63 (m, 3H); 7,93 (t, $J$ = 7,8 Hz, 1H); 8,03 (d, $J$ = 7,8 Hz, 1H), 9,24 (br m, 1H).

**Beispiel 4**

***N*-(4-Fluorphenyl)-6-[3-(trifluormethyl)phenoxy]pyridin-2-carboxamid**

Es wurde verfahren wie in Beispiel 3 beschrieben, jedoch wurde anstelle von Palladium/Aktivkohle 17,5 mg (25 µmol) Dichloro-bis(triphenylphosphin)palladium(II) eingesetzt. Der CO-Druck betrug 12,8 bar, die Temperatur 150 °C und die Reaktionsdauer 17,7 h. Mittels GC wurde die Zusammensetzung der gelösten Produkte in der Xylol-Phase bestimmt Gefunden wurden 96,0% Titelverbindung (Amid) und 4,0% Nebenprodukte (2,3% sekundäres Amin, 1,7% Hydrogenolyseprodukt).

**Beispiel 5**

***N*-(4-Fluorphenyl)-6-[3-(trifluormethyl)phenoxy]pyridin-2-carboxamid**

Es wurde verfahren wie in Beispiel 4 beschrieben, jedoch wurde anstelle des 1,4-Bis(diphenylphosphino) butans die gleiche molare Menge 1,3-Bis(diphenytphosphino)propan (IV, $n$ = 3, $R^8 = R^9 = R^{10} = R^{11}$ = Phenyl) eingesetzt. Der CO-Druck betrug 16 bar, die Reaktionsdauer 21,6 h Mittels GC wurde die Zusammensetzung der gelösten Produkte in der Xylol-Phase bestimmt. Gefunden wurden 98,9% Titelverbindung (Amid), 0,1% Edukt und 1,0% Nebenprodukte (0,3% sekundäres Amin und 0,7% Hydrogenolyseprodukt).

**Beispiel 6**

***N*-(2,4-Difluorphenyl)-2-[3-(trifluoromethyl)phenoxy]pyridin-3-carboxamid**

(Diflufenicam)

Analog zu Beispiel 4 wurden 6,84 g (25 mmol) 3-Chlor-2-(3-trifluormethyl)phenoxypyridin (hergestellt nach Beispiel 2), 4,84 g (37,5 mmol) 2,4-Difluoranilin, 2,92 g (27,5 mmol) Natriumcarbonat, 17,5 mg (25 µmol) Dichloro-bis (triphenylphosphin)palladium(II) und 0,32 g (0,75 mmol) 1,4-Bis(diphenylphosphino)butan in 25 ml Xylol unter 15 bar CO-Druck bei 190-195 °C 19 h umgesetzt Der Umsatz war ca. 80%. Die Aufarbeitung wurde wie in Beispiel 3 durchgefuhrt Es wurden 6 g Rohprodukt als gelber kristalliner Feststoff erhalten. Zur Reinigung wurde aus 50 ml Methylcyclohexan umkristallisiert.
Ausbeute 3,25 g (33%) weisser Feststoff
Schmp.. 157-159 °C

MS, *m z* 394 (M'), 266 (100%)

$^1$H NMR ($CDCl_3$): $\delta$ = 6,89-6,96 (m, 2H), 7,26 (m, 1H); 7,46 (m, 1H); 7,54-7,63 (m, 3H), 8,28 (dd; 1 H); 8,52 (m, 1H); 8,71 (dd, 1H); 9,97 (br. s, 1H).

**Vergleichsbeispiel 1**

Es wurde verfahren wie in Beispiel 4 beschrieben, jedoch wurde anstelle von 1,4-Bis(diphenylphosphino) butan die gleiche molare Menge Triphenylphosphin eingesetzt. Nach einer Reaktionsdauer von 15,5 h bei 15 bar CO-Druck wurde mittels GC die Zusammensetzung der gelösten Produkte in der Xylol-Phase bestimmt. Gefunden wurden nur 43,2% des gewünschten Produkts, aber 56,8% unumgesetztes Edukt.

**Vergleichsbeispiel 2**

Es wurde verfahren wie in Beispiel 4 beschrieben, jedoch wurde anstelle von 1,4-Bis(diphenylphosphino) butan die gleiche molare Menge Tri-*n*-butylphosphin eingesetzt. Nach einer Reaktionsdauer von 15 h bei 14 bar CO-Druck wurde mittels GC die Zusammensetzung der gelösten Produkte in der Xylol-Phase bestimmt. Gefunden wurden nur Spuren (0,4%) des gewünschten Produkts, aber 96,8% unumgesetztes Edukt.

**Vergleichsbeispiel 3**

Es wurde verfahren wie in Beispiel 4 beschrieben, jedoch wurde anstelle von 1,4-Bis(diphenylphosphino) butan die gleiche molare Menge 1,2-Bis(diphenylphosphino)ethan eingesetzt. Nach einer Reaktionsdauer von 20,2 h bei 14,7 bar CO-Druck wurde mittels GC die Zusammensetzung der gelösten Produkte in der Xylol-Phase bestimmt. Gefunden wurden nur Spuren (2,2%) des gewünschten Produkts, aber 97,7% unumgesetztes Edukt.

## Patentansprüche

**1.** Verfahren zur Herstellung von Amiden der allgemeinen Formel

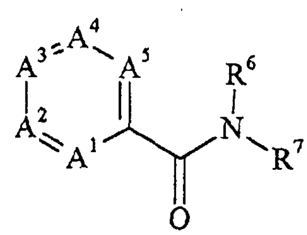

I,

worin

$A^1$ Stickstoff oder $CR^1$,
$A^2$ Stickstoff oder $CR^2$,
$A^3$ Stickstoff oder $CR^3$,
$A^4$ Stickstoff oder $CR^4$,
und $A^5$ Stickstoff oder $CR^5$ ist,

mit der Massgabe, dass wenigstens eines der Ringglieder $A^1$ bis $A^5$ Stickstoff ist und nicht zwei Stickstoffatome unmittelbar miteinander verbunden sind,

$R^1$ bis $R^5$, soweit vorhanden, unabhängig voneinander Wasserstoff $C_{1-4}$-Alkyl, Phenyl, Naphthyl, Biphenylyl oder Anthracenyl sind, wobei einer der Substituenten $R^1$ bis $R^5$ eine Gruppe der Formel -OR ist, in welcher R gegebenenfalls mit Halogen, Methyl, Trifluormethyl, Methoxy, Methylthio und/oder Ethansulfonyl substituiertes Phenyl, Naphthyl, Biphenylyl, Anthracenyl, Furyl, Pyrrolyl, Pyrazolyl, Thiophenyl, Pyridyl, Indolyl oder Chinolinyl ist,

$R^6$ Wasserstoff oder $C_{1-4}$-Alkyl
und $R^7$ gegebenenfalls wie oben substituiertes Phenyl, Naphthyl, Biphenylyl, Anthracenyl, Furyl, Pyrrolyl, Pyrazolyl, Thiophenyl, Pyridyl, Indolyl oder Chinolinyl ist, **dadurch gekennzeichnet, dass** eine Halogenverbindung der allgemeinen Formel

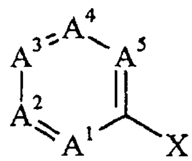

II,

worin $A^1$ bis $A^5$ die oben genannten Bedeutungen haben und X Chlor, Brom oder Iod ist, mit Kohlenmonoxid und einem Amin der allgemeinen Formel

$$R^6\text{-NH-}R^7 \qquad \text{III,}$$

worin $R^6$ und $R^7$ die oben genannten Bedeutungen haben, in Gegenwart eines *in situ* aus einem Pd(II)-Komplex mit einem Diphosphin der allgemeinen Formel

$$R^8R^9P\text{—}[CH_2]_n\text{—}PR^{10}R^{11} \qquad \text{IV,}$$

worin $R^8$ bis $R^{11}$ unabhängig voneinander Phenyl oder wie oben substituiertes Phenyl bedeuten und *n* 3 oder 4 ist, gebildeten Komplexes
und einer Base zur Reaktion gebracht wird.

**2.** Verfahren nach Anspruch 1, **dadurch gekennzeichnet, dass** $A^2$ Stickstoff und Teil eines Pyridinringes ist.

**3.** Verfahren nach Anspruch 2, **dadurch gekennzeichnet, dass** $R^1$ eine Gruppe der Formel - OR ist, wobei R die in Anspruch 1 genannte Bedeutung hat.

**4.** Verfahren nach Anspruch 1, **dadurch gekennzeichnet, dass** $A^1$ Stickstoff und Teil eines Pyridinringes ist.

**5.** Verfahren nach Anspruch 1, **dadurch gekennzeichnet, dass** $A^1$ und $A^5$ Stickstoff und Teil eines Pyrimidinringes sind.

**6.** Verfahren nach Anspruch 1, **dadurch gekennzeichnet, dass** $A^1$ und $A^4$ Stickstoff und Teil eines Pyrazinringes sind.

**7.** Verfahren nach Anspruch 1, **dadurch gekennzeichnet, dass** $A^1$, $A^3$ und $A^5$ Stickstoff sind.

**8.** Verfahren nach Anspruch 4, 5, 6 oder 7, **dadurch gekennzeichnet, dass** $R^2$ eine Gruppe der Formel -OR ist, wobei R die in Anspruch 1 genannte Bedeutung hat.

**9.** Verfahren nach Anspruch 3 oder 8, **dadurch gekennzeichnet, dass** R eine gegebenenfalls substituierte Phenylgruppe ist.

**10.** Verfahren nach einem der Ansprüche 1 bis 9, **dadurch gekennzeichnet, dass** $R^6$ Wasserstoff und $R^7$ eine gegebenenfalls substituierte Phenylgruppe ist.

**11.** Verfahren nach einem der Ansprüche 1 bis 10, **dadurch gekennzeichnet, dass** X Chlor ist.

**12.** Verfahren nach einem der Ansprüche 1 bis 11, **dadurch gekennzeichnet, dass** als Diphosphin (IV) 1,3-Bis(diphenylphosphino)propan oder 1,4-Bis(diphenylphosphino)butan eingesetzt wird.

## Claims

1. Process for the preparation of amides of the general formula

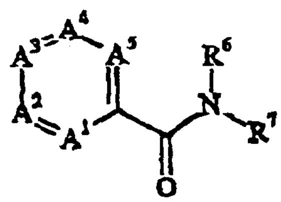

I,

wherein

$A^1$ is nitrogen or $CR^1$,
$A^2$ is nitrogen or $CR^2$,
$A^3$ is nitrogen or $CR^3$,
$A^4$ is nitrogen or $CR^4$,
and $A^5$ is nitrogen or $CR^5$,

with the proviso that at least one of the ring members $A^1$ to $A^5$ is nitrogen and two nitrogen atoms are not bonded directly to one another,

$R^1$ to $R^5$, where present, independently of one another are hydrogen, $C_{1-4}$-alkyl, phenyl, naphthyl, biphenylyl or anthracenyl, wherein one of the substituents $R^1$ to $R^5$ is a group of the formula -OR, in which R is phenyl, naphthyl, biphenylyl, anthracenyl, furyl, pyrrolyl, pyrazolyl, thiophenyl, pyridyl, indolyl or quinolinyl, optionally substituted by halogen, methyl, trifluoromethyl, methoxy, methylthio and/or ethanesulfonyl,
$R^6$ is hydrogen or $C_{1-4}$-alkyl
and $R^7$ is phenyl, naphthyl, biphenylyl, anthracenyl, furyl, pyrrolyl, pyrazolyl, thiophenyl, pyridyl, indolyl or quinolinyl, optionally substituted as above, **characterized in that** a halogen compound of the general formula

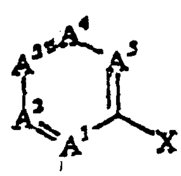

II,

wherein $A^1$ to $A^5$ have the above-mentioned meanings and X is chlorine, bromine or iodine, is reacted with carbon monoxide and an amine of the general formula

$$R^6\text{-NH-}R^7 \qquad \text{III},$$

wherein $R^6$ and $R^7$ have the above-mentioned meanings, in the presence of a complex formed *in situ* from a Pd (II) complex with a diphosphine of the general formula

$$R^8R^9P\text{-} [CH_2]_n\text{-}PR^{10}R^{11} \qquad \text{IV},$$

wherein $R^8$ to $R^{11}$ independently of one another denote phenyl or phenyl substituted as above and $n$ is 3 or 4, and a base.

2. Process according to claim 1, **characterized in that** $A^2$ is nitrogen and is part of a pyridine ring.

3. Process according to claim 2, **characterized in that** $R^1$ is a group of the formula -OR, wherein R has the meaning given in claim 1.

4. Process according to claim 1, **characterized in that** $A^1$ is nitrogen and is part of a pyridine ring.

5. Process according to claim 1, **characterized in that** $A^1$ and $A^5$ are nitrogen and are part of a pyrimidine ring.

6. Process according to claim 1, **characterized in that** $A^1$ and $A^4$ are nitrogen and are part of a pyrazine ring.

7. Process according to claim 1, **characterized in that** $A^1$, $A^3$ and $A^5$ are nitrogen.

8. Process according to claim 4, 5, 6 or 7, **characterized in that** $R^2$ is a group of the formula -OR, wherein R has the meaning given in claim 1.

9. Process according to claim 3 or 8, **characterized in that** R is an optionally substituted phenyl group.

10. Process according to one of claims 1 to 9, **characterized in that** $R^6$ is hydrogen and $R^7$ is an optionally substituted phenyl group.

11. Process according to one of claims 1 to 10, **characterized in that** X is chlorine.

12. Process according to one of claims 1 to 11, **characterized in that** 1,3-bis(diphenylphosphino)propane or 1,4-bis (diphenylphosphino)butane is employed as the diphosphine (IV).

**Revendications**

1. Procédé pour la préparation d'amides de la formule générale

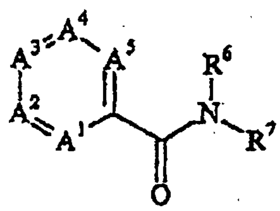

I,

dans laquelle,

$A^1$ est l'azote ou $CR^1$,
$A^2$ est l'azote ou $CR^2$,
$A^3$ est l'azote ou $CR^3$,
$A^4$ est l'azote ou $CR^4$,
et $A^5$ est l'azote ou $CR^5$,

sous réserve qu'au moins l'un des chaînons de cycle $A^1$ à $A^5$ soit l'azote et que deux atomes d'azote ne soient pas liés directement entre eux,

$R^1$ à $R^5$, s'ils sont présents, sont indépendamment l'un de l'autre l'hydrogène, un groupe alkyle en $C_{1-4}$, phényle, naphtyle, biphénylyle ou anthracényle, tandis que l'un des substituants $R^1$ à $R^5$ est un groupe de la formule -OR, dans laquelle R est un groupe phényle, naphtyle, biphénylyle, anthracényle, furyle, pyrrolyle, pyrazolyle, thiophényle, pyridyle, indolyle ou quinolinyle, éventuellemnt substitué par halogène, méthyle, trifluorométhyle, méthoxy, méthylthio et/ou éthanesulfonyle,
$R^6$ est l'hydrogène ou un alkyle en $C_{1-4}$
et $R^7$ est un groupe phényle, naphtyle, biphénylyle, anthracényle, furyle, pyrrolyle, pyrazolyle, thiophényle, pyridyle, indolyle ou quinolinyle éventuellement substitué comme ci-dessus, **caractérisé en ce qu'**un composé halogène de la formule générale

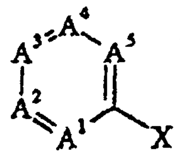

II,

dans laquelle $A^1$ à $A^5$ ont les significations ci-dessus et X est le chlore, le brome ou l'iode, est mis à réagir avec le monoxyde de carbone et une amine de la formule générale

$$R^6\text{-NH-}R^7 \quad \text{III,}$$

dans laquelle $R^6$ et $R^7$ ont les significations précitées, avec une base et en présence d'un complexe formé *in situ* à partir d'un complexe Pd(II) avec une disphosphine de la formule générale

$$R^8R^9P—[CH_2]_n—PR^{10}R^{11} \quad \text{IV,}$$

dans laquelle $R^8$ à $R^{11}$ signifient indépendamment l'un de l'autre un groupe phényle ou phényle substitué comme ci-dessus et *n* est égal à 3 ou 4.

2. Procédé selon la revendication 1, **caractérisé en ce que** $A^2$ est l'azote et une partie d'un cycle pyridine.

3. Procédé selon la revendication 2, **caractérisé en ce que** $R^1$ est un groupe de la formule -OR, dans laquelle R a la signification citée dans la revendication 1.

4. Procédé selon la revendication 1, **caractérisé en ce que** $A^1$ est l'azote et une partie d'un cycle pyridine.

5. Procédé selon la revendication 1, **caractérisé en ce que** $A^1$ et $A^5$ sont l'azote et une partie d'un cycle pyrimidine.

6. Procédé selon la revendication 1, **caractérisé en ce que** $A^1$ et $A^4$ sont l'azote et une partie d'un cycle pyrazine.

7. Procédé selon la revendication 1, **caractérisé en ce que** $A^1$, $A^3$ et $A^5$ sont l'azote.

8. Procédé selon la revendication 4, 5, 6 ou 7, **caractérisé en ce que** $R^2$ est un groupe de la formule -OR, tandis que R a la signification citée dans la revendication 1.

9. Procédé selon la revendication 3 ou 8, **caractérisé en ce que** R est un groupe phényle éventuellement substitué.

10. Procédé selon l'une des revendications 1 à 9, **caractérisé en ce que** $R^6$ est l'hydrogène et $R^7$ est un groupe phényle éventuellement substitué.

11. Procédé selon l'une des revendications 1 à 10, **caractérisé en ce que** X est le chlore.

12. Procédé selon l'une des revendications 1 à 11, **caractérisé en ce qu'**en tant que diphosphine(IV), on utilise le 1,3-bis(diphénylphosphino)propane ou le 1,4-bis(diphénylphosphino)butane.